# EUROPEAN PATENT SPECIFICATION

(11) **EP 2 219 584 B1**
(45) Date of publication and mention of the grant of the patent: **13.06.2012**
(21) Application number: 08868673.8
(22) Date of filing: 09.12.2008
(51) Int. Cl.: A61J 15/00, A61B 17/00, A61F 2/00, A61F 5/00, A61M 25/02, A61M 39/02, A61M 39/20

(54) **DESIGNER ACCESSORY FOR USE WITH AN INTRACORPOREAL MEDICAL DEVICE**
DESIGNERZUBEHÖRTEIL ZUR VERWENDUNG MIT EINER INTRAKORPORALEN MEDIZINISCHEN VORRICHTUNG
ACCESSOIRE DÉCORATIF DESTINÉ À ÊTRE UTILISÉ AVEC UN DISPOSITIF MÉDICAL INTRACORPOREL

(30) Priority: 21.12.2007 US 4657
(43) Date of publication of application: 25.08.2010
(73) Proprietor: KIMBERLY-CLARK WORLDWIDE, INC., Neenah, WI 54956 (US)
(72) Inventor: O'BRIEN, Nicole, Sams, Marietta, GA 30066 (US); MCMICHAEL, Donald, Jay, Roswell Georgia 30076 (US); BACON, John, Kenneth, Provo Utah 84604 (US)
(74) Representative: Davies, Christopher Robert
(86) International application number: PCT/IB2008/055178
(87) International publication number: WO 2009/083835

(56) References cited:
- WO-A-02/066108
- US-A- 5 549 657
- US-B1- 6 438 758

## Description

### BACKGROUND

Catheterization techniques for providing nutritional solutions directly into the stomach or intestines of a patient are known. These techniques form a stoma in the stomach or intestinal wall of the patient through which a catheter or other intracorporeal medical device is placed. Feeding solutions may then be injected through the catheter inserted in the stomach to provide nutrients directly to the stomach or intestines. This process is known as enteral feeding.

In many instance, these intrecorporeal medical devices are configured to have a low profile above a patient's skin so that the intracorporeal medical devices do not interfere with the patient's other activities. This is a particularly important feature for both active teenagers and smaller children, because these intrecorporeal medical devices often become a permanent part of a patient's life.

Despite the advantageous low profile of many of these devices, many of these children are hesitant to use or even refuse to use them because of fear or lingering insecurities about the devices. Many of these child patients long for a feeling of normalcy in their lives and they believe the medical nature of these devices diminishes that.

Because these intracorporeal medical devices provide a vital role in providing nutrition to child patients who otherwise might not be able to provide it for themselves, it is imperative that oil children who need these intracorporeal medical devices actually use them. US 5 549 657 discloses a low profile adaptor for gastronomy feeding tube.

Thus, there remains a need for device that will change a child's perception of these intracorporeal medical devices from as being scary and medical into something fun, attractive, and customizable, without compromising the functionality of the intracorporeal medical device itself.

### SUMMARY OF INVENTION

The present invention provides for an apparatus for covering an intracorporeal medical device in accordance with claim 1. The apparatus includes an anchoring body having a top surface, a first side surface, and a second side surface. The apparatus may be manufactured utilizing flexible material polyurethane, urethane, silicone, or combinations thereof. The intracorporeal medical device which is covered by the apparatus includes a top surface, a first side surface, a second side surface, a bottom surface, a tether, a central opening, and a shaft which is inserted into an artificial opening during use.

The anchoring body of the apparatus is designed to be in frictional communication with a top surface, a first side surface, and a second side surface of an intracorporeal medical device and will desirably be substantially the same shape as the top surface, the first side surface, and the second side surface of the intracorporeal medical device. Additionally, the anchoring body defines an opening for insertion of the tether into the central opening of the intracorporeal medical device.

The apparatus includes a hinged cover connected to the anchoring body by a connector. The hinged cover Is adapted to be in communication with and surround the tether so that the tether and the central opening are not exposed to the outside environment after insertion of the tether into the central opening of the medical device. This arrangement provides added protection to the tether and central opening because it may decrease the amount of bacteria congregating around the tether and central opening.

Further, while the apparatus is covering the intracorporeal medical device, the apparatus covers only the top surface, first side surface, and second side surface of the intracorporeal device, but does not cover the bottom surface or shaft of the intracorporeal medical device. This is an important feature because it prevents the portions of the intracorporeal medical device which are in communication with the patient (the bottom surface and shaft) from being contaminated with any bacteria that may be present on the top surface, first side surface, and second side surface of the apparatus. Further, it is an important feature because images may be present on the apparatus and the presence of these images on the bottom surface or shaft of the intracorporeal medical device could entice a patient to manipulate and or remove the device in order to view the images. In this regard, patients using the apparatus should not have a need to manipulate the device, before, during, or after use, making the apparatus desirably a hands-free device for the patient.

As previously discusses, one or more images may be formed on the apparatus. These images may desirably have favorable psychological associations for the patient and may be formed in two dimensions or three dimensions on the anchoring body. Desirably, these psychologically favorable images will encourage a patient to use the intracorporeal medical device and will reduce fear regarding the use of the intracorporeal medical device.

The invention may provide for a system for attaching a customized decorative cover to an intracorporeal medical device. The customized decorative cover includes an anchoring body having a top surface, a first side surface, and a second side surface. The intracorporeal medical device which is covered by the customized decorative cover includes a top surface, a first side surface, a second side surface, a bottom surface, a tether, a central opening, and a shaft which is inserted into an artificial opening during use.

The anchoring body of the customized decorative cover is designed to be in fictional communication with a top surface, a first side surface, and a second side surface of an intracorporeal medical device and will desirably be substantially the same shape as the top surface, the first side surface, and the second side surface of the intracorporeal medical device. Additionally, the anchoring body defines an opening for insertion of the tether into the central opening of the intracorporeal medical device.

Further, the customized decorative cover includes a hinged cover connected to the anchoring body by a connector. The hinged cover is adapted to be in communication with and surround the tether so that the tether and the central opening are not exposed to the outside environment after insertion of the tether into the central opening of the medical device.

### BRIEF DESCRIPTION OF DRAWINGS

Fig 1A is a perspective view of the hinged cover of the apparatus surrounding the tether of the intracorporeal medical device.
Fig. 1 B is a perspective view of the hinged cover of the apparatus pulled back to reveal a tether inserted into the central opening of the medical device.
Fig. 2 is a perspective view of an intracorporeal medical device adapted to be covered by the apparatus.
Fig. 3 is a perspective view of the apparatus/removable cover attached to an intracorporeal medical device.
Fig. 4 is a sectional view of the apparatus attached to the intracorporeal medical device.
Fig. 5 shows examples of images which may be formed on the apparatus.
Fig. 6 is a perspective view of an apparatus adapted to cover an intracorporeal medical device.

### DEFINITIONS

Artificial Opening- An opening into the human body which is not naturally found on the human body. Artificial Openings include openings formed by surgical methods and formed by devices such as catheters. These artificial openings include, for example, stoma sites. Naturally occurring body openings include the mouth, ears, reproductive, and anal openings.

Intracorporeal Medical Device- A device which is inserted into an artificial opening to facilitate transmission of fluids into the human body or removal of fluids from the human body through the artificial opening. These devices include, but are not limited to, gastronomy tubes, ostomy tubes, feeding tubes, and enteral feeding adapters. Generally, upon insertion, of an intracorporeal medical device into an artificial body opening, a portion of the intracorporeal medical device remains outside the body.

### DETAILED DESCRIPTION

The apparatuses of the present invention provide for removable covers having images thereon which encourage a patient to use a medical device. These covers are in communication with the medical device, but do not interfere with the use of the intracorporeal medical device.

The invention will be described with reference to the following description and figures which illustrate certain embodiments. It will be apparent to those skilled in the art that these embodiments do not represent the full scope of the invention which is broadly applicable in the form of variations and equivalents as may be embraced by the claims appended hereto. Furthermore, features described or illustrated as part of one embodiment may be used with another embodiment to yield still a further embodiment. It is intended that the scope of the claims extend to all such variations and embodiments.

In the interests of brevity and conciseness, any ranges of values set forth in this specification contemplate all values within the range and are to be construed as support for claims reciting any sub-ranges having endpoints which are whole number values within the specified range in question. By way of a hypothetical illustrative example, a disclosure in this specification of a range of from 1 to 5 shall be considered to support claims to any of the following ranges: 1-5; 1-4; 1-3; 1-2; 2-5; 2-4; 2-3; 3-5; 3-4; and 4-5.

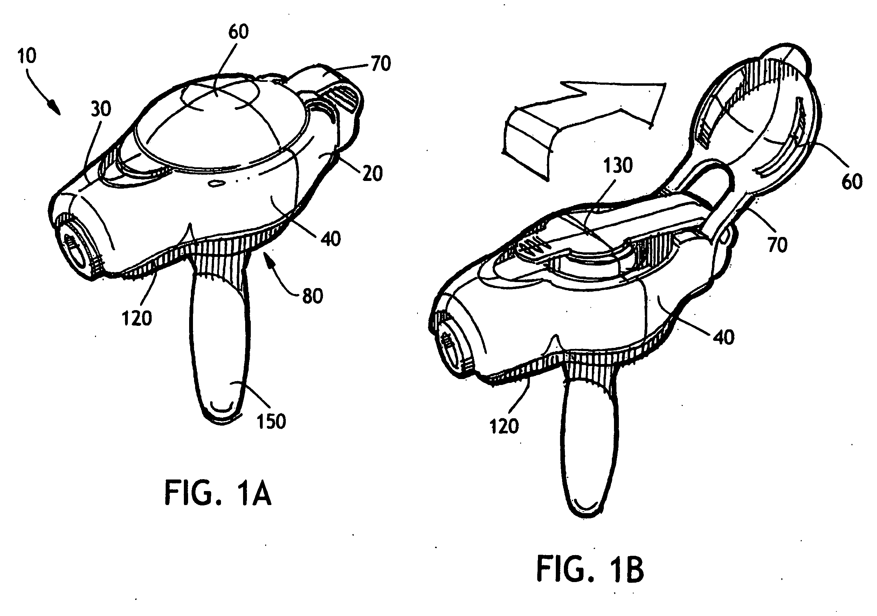

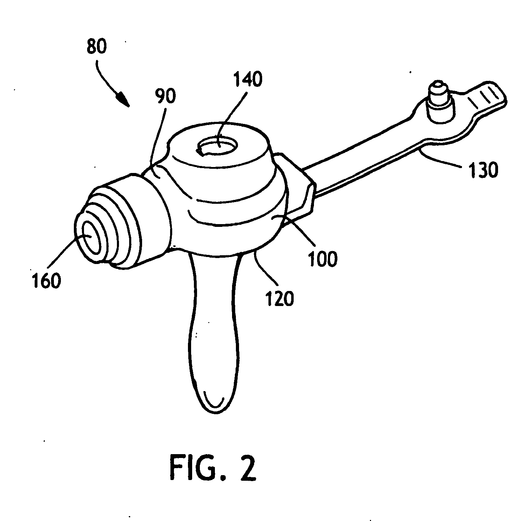

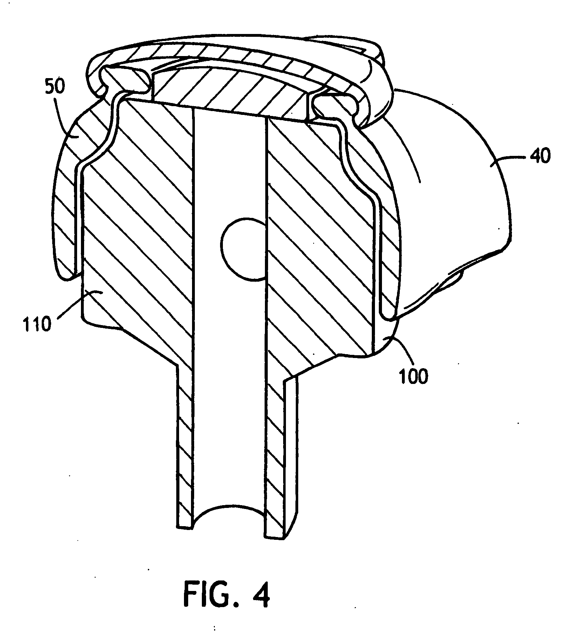

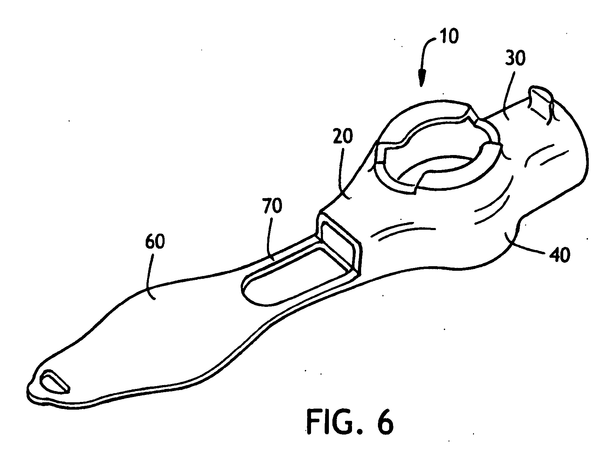

Referring to Fig. 1 A, 1B, 3, 4, and 6, an apparatus 10 for covering an intracorporeal medical device 80 is provided. The apparatus is a removable cover having an anchoring body 20 which has a top surface 30, a first side surface 40, and a second side surface 50. Desirably, upon placement of the apparatus on the intracorporeal medical device (by hand or through other mechanisms), the top surface 30, first side surface 40, and second side surface 50 (See Figs 1A and 1B) of the apparatus will be in communication with the top surface 90, first side surface 100, and second side surface 110 of the intracorporeal medical device (See Fig. 2). Thus, the apparatus, upon covering the medical device, will have substantially the same shape as the medical device. In this regard, the relationship between the apparatus and the intracorporeal medical device is analogous to the relationship between a glove and hand.

The apparatus may be made from any flexible material which allows the apparatus to be in communication with the intracorporeal medical device and remain in place on the device before, during, and after use. These materials include, but are not limited to, polyurethane, urethane, silicone, or combinations thereof. The apparatus itself may be manufactured utilizing these materials, by any method known generally in the art for manufacturing flexible material. These methods include various plastic molding techniques, for example, which are known in the art. Additionally, many other techniques for manufacturing flexible material are known in the art and could be readily utilized to manufacture the apparatus of the current invention.

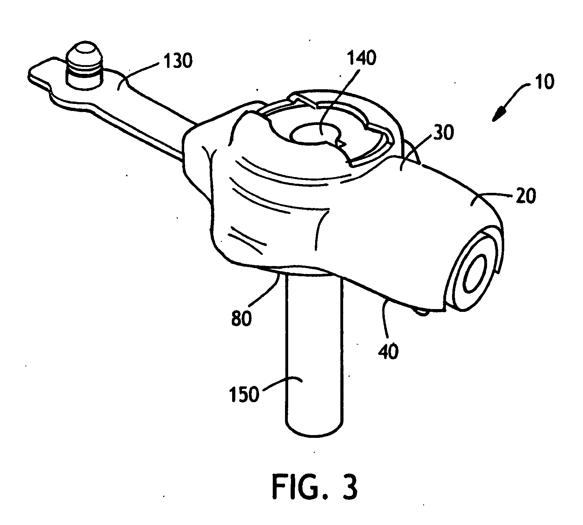

Returning to Fig. 3, the apparatus includes an opening in its top surface 30. The opening allows the tether 130 of the intracorporeal medical device to plug the central opening 140 of the medical device when the device is not in use.

Turning to Figs. 1A and 1B, the apparatus may also include a hinged cover 60 which is connected to the anchoring body 20 by a connector 70. Desirably, the dimensions of the hinged cover will correspond with the dimensions of the tether and will be adapted to be in communication with the tether upon insertion of the tether into the central opening of the intracorporeal medical device. The hinged cover may remain in communication with the tether through various mechanisms, including, but not limited to, application of an adhesive onto the hinged cover and/or tether; and fastening the hinged cover to the tether or to the anchoring body. Advantageously, the use of the hinged cover in communication with the tether may desirably provide additional protection to the tether and central opening because it may decrease the amount of bacteria congregating around the tether and central opening when the tether is plugged into the central opening.

Turning to Fig. 2, in practice the shaft 150 of the intracorporeal medical device, desirably an enteral feeding device, will be inserted through a stoma on the patient and held in place by a balloon which is inflated by a balloon port 160. After insertion of the device a feeding tube will desirably be inserted through the central opening 140 of the medical device and fluid will be introduced into the patient's stomach to nourish the patient. As shown in Fig. 1B, after use, the tether of the medical device is inserted into the central opening to prevent dirt and bacteria from traveling through the central opening and shaft into the patient's body. Low Profile Enteral Feeding Devices appropriate for use with the present invention are detailed in, for example, U.S. Pat. No. 6,808,521.

Advantageously, the apparatus does not interfere with the essential function of the intracorporeal medical device when it is placed in communication with the intracorporeal medical device. In this regard, during use of the intracorporeal medical device, the apparatus does not impede the inflation of the balloon holding the enteral feeding device in place within the body because the balloon port 160 is unobstructed. Additionally, the apparatus does not interfere with access to the central opening through which the feeding tube is inserted. Further, the apparatus does not touch the skin or bottom surface of the intracorporeal medical device and is not in communication with the patient's skin. This is important because if the apparatus were in communication with the patient's skin it could increase the susceptibility of the stoma site to irritation and infection.

Although the apparatus does not interfere with the use of the feeding device, and in some cases, it actually enhances the utility of the feeding device, for example, when utilizing the hinged cover, further enhancements to the apparatus may be desirable in particular situations to achieve a desired goal.

For example, in order to encourage and facilitate the use of the intracorporeal medical device for child and teenage patients, images may be included upon the apparatus. Desirably, these images may be included on any part of the apparatus including the anchoring body, first side, second side, connector, or hinged cover. The images may be imparted onto the apparatus by a variety of methods known to those of ordinary skill in the art. These methods, include, but are not limited to, printing and lamination. Additionally, many other techniques known in the art could be readily utilized to impart images on the flexible material of the apparatus.

Regardless of the method of imparting images on the apparatus, the images utilized are designed to invoke favorably psychological association and good feelings in order to encourage the patient to use the device and reduce fear regarding the use of the medical device.

These images may be represented in two or in three dimensions upon the apparatus. Further, these images may be customized based on various demographics, for example, age, gender, cultural background, medical condition, hobbies, sports, and personal interests.

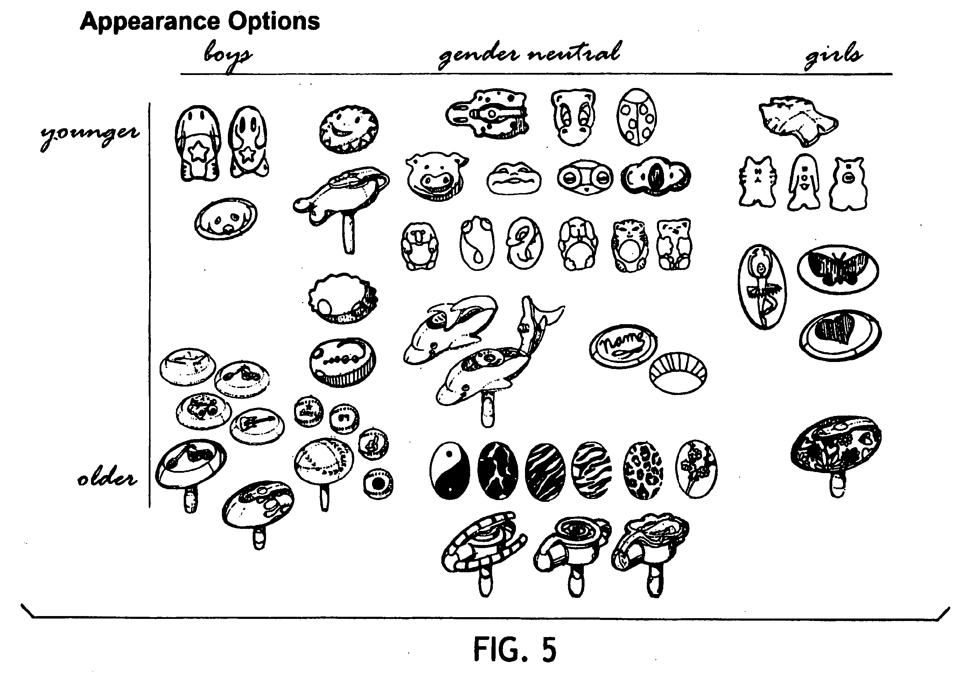

Non-limiting examples of suitable images are illustrated in Fig. 5. These examples are arranged by age and gender. These examples include animals, vehicles, cartoon characters, flowers, sports mascots, airplanes, ballerinas, and name tags. Further non-limiting examples of suitable images may include a representation of an actual or fictional person, a superhero, or a musician. Additionally, inspirational, religious, or humorous images or messages may be represented upon the apparatus. Regardless of the type of image or images selected, the image should be customized for the particular user and have favorable psychological association for him or her.

In addition to apparatus provided above, the present invention encompasses a system for attaching a customized decorative cover to an intracorporeal medical device.

Generally speaking, the system includes a customized decorative cover having an anchoring body which has a top surface, a first side surface, and a second side surface. The customized decorative cover covers an intracorporeal medical device having a top surface, a first side surface, a second side surface, a bottom surface, a tether, a central opening, and a shaft which is inserted into an artificial opening during use.

The anchoring body of the customized decorative cover is designed to be in frictional communication with a top surface, a first side surface, and a second side surface of an intracorporeal medical device and will desirably be substantially the same shape as the top surface, the first side surface, and the second side surface of the intracorporeal medical device. Additionally, the anchoring body defines an opening for insertion of the tether into the central opening of the intracorporeal medical device.

The system further includes a hinged cover connected to the anchoring body by a connector. The hinged cover is adapted to be in communication with and surround the tether so that the tether and the central opening are not exposed to the outside environment after insertion of the tether into the central opening of the medical device.

Additionally, the system includes a means for attaching the anchoring body to the intracorporeal medical device, and a means for attaching the hinged cover to the tether of the intracorporeal medical device. Means for attaching the anchoring body to the intracorporeal medical device include, but are not limited to, hands. Means for attaching the hinged cover to the tether of the medical device include, but are not limited to, the use of adhesives or fasteners.

## Claims

1. An apparatus (10) for covering an intracorporeal medical device (80) having a top surface a first side surface (100), a second side surface (110), a bottom surface, a tether (130), a central opening (140), and a shaft (150), said intracorpored medical device being insertable into an artificial opening to facilitate transmission of fluids into the human body or removal of fluids from the human body through the artificial opening, the apparatus comprising:
An anchoring body (20) having a top surface (30), a first side surface (40), and a second side surface (50), the anchoring body adapted to be in frictional communication with a top surface (90), a first side surface (100), and a second side surface (110) of on intracorporeal medical device, **characterised In that** the anchoring body (20) defines an opening for insertion of a tether (130) into a central opening (140) of the intracorporeal medical device;
and a hinged cover (60) connected to the anchoring body by a connector (70), the hinged cover being adapted to be in communication with and surround the tether (130) so that the tether and tho central opening are not exposed to the outside environment after insertion of the tether into the central opening (140) of the medical device.

2. the apparatus of claim 1, wherein, during use, a shaft (150) of the intracorporeal medical device is inserted into an artificial opening.

3. The apparatus of claim 2, wherein the anchoring body (20) is not in communication with a bottom surface of the intracorporeal medical device, nor In communication with the shaft (150) of the intracorporeal medical device inserted into the artificial opening, further wherein the bottom surface of the intracorporeal medical device is in communication with the patient during use and wherein the anchoring body is not in communication with the patient during use.

4. The apparatus of any preceding claim, wherein the apparatus comprises at least one image with which the patient has favorable psychological associations, the image being adapted to encourage a patient to use the intracorporeal medical device and reduce fear regarding the use of the intracorporeal medical device.

5. The apparatus of claim 4, wherein the at least one image is represented in two dimensions on at least one surface of the anchoring body (20).

6. The apparatus of claim 4, wherein the at least one image is represented in three dimensions on at least one surface of the anchoring body (20).

7. The apparatus of any preceding claim, wherein the apparatus comprises polyurethane, urethane, silicone, or a combination thereof.

8. The apparatus of any preceding claim, wherein the anchoring body (20) covers the top surface, the first side surface and the second side surface of the medical device and, upon covering the medical device, the anchoring body is substantially the same shape as the top surface, the first side surface, and the second side surface of the medical device.

9. The apparatus of claim 1, wherein the apparatus is a removable cover.

10. A system for attaching a customized decorative cover to an intracorporeal medical device having a top surface a first side surface, a second side surface, a bottom surface, a tether, a central opening, and a shaft, the system comprising the apparatus of claim 1, a means for attaching the anchoring body to the intracorporeal medical device; and a means for attaching the hinged cover to the tether of the intracorporeal medical device.

## Patentansprüche

1. Gerät (10) zum Abdecken einer intrakorporalen medizinischen Vorrichtung (80), welche eine obere Oberfläche, eine erste Seitenoberfläche (100), eine zweite Seitenoberfläche (110), einen Bodenoberfläche, ein Band (130), eine zentrale Öffnung (140) und einen Schaft (150) aufweist, wobei das Gerät umfasst:
einen Verankerungskörper (20) mit einer oberen Oberfläche (30), einer ersten Seitenoberfläche (40) und einer zweiten Seitenoberfläche (50), wobei der Verankerungskörper geeignet ist, in reibschlüssiger Verbindung zu stehen mit einer oberen Oberfläche (90), einer ersten Seitenoberfläche (100) und einer zweiten Seitenoberfläche (110) einer intrakorporalen medizinischen Vorrichtung, **dadurch gekennzeichnet, dass** der Verankerungskörper (20) eine Öffnung definiert zum Einführen eines Bands (130) in eine zentrale Öffnung (140) der intrakorporalen medizinischen Vorrichtung;
eine Scharnierabdeckung (60), welche mit dem Verankerungskörper über einen Verbinder (70) verbunden ist, wobei die Scharnierabdeckung geeignet ist, in Verbindung mit dem Band (130) zu stehen und dieses zu umgeben, so dass das Band und die zentrale Öffnung nach dem Einführen des Bands in die zentrale Öffnung (140) der medizinischen Vorrichtung nicht der äußeren Umgebung ausgesetzt sind.

2. Gerät gemäß Anspruch 1, wobei, in Verwendung, ein Schaft (150) der intrakorporalen medizinischen Vorrichtung in eine künstliche Öffnung eingeführt ist.

3. Gerät gemäß Anspruch 2, wobei der Verankerungskörper (20) weder in Verbindung mit einer Bodenoberfläche der intrakorporalen medizinischen Vorrichtung noch in Verbindung mit dem Schaft (150) der intrakorporalen medizinischen Vorrichtung steht, welche in die künstliche Öffnung eingeführt ist, wobei des Weiteren die Bodenoberfläche der intrakorporalen medizinischen Vorrichtung während der Verwendung in Verbindung mit dem Patienten steht, und wobei der Verankerungskörper während der Verwendung nicht in Verbindung mit dem Patienten steht.

4. Gerät gemäß einem der vorherigen Ansprüche, wobei das Gerät mindestens ein Bild aufweist, mit welchem der Patient positive psychologische Assoziationen verbindet, wobei das Bild geeignet ist, einen Patienten dazu anzuregen, die intrakorporale medizinische Vorrichtung zu verwenden, und Angst hinsichtlich der Verwendung der intrakorporalen medizinischen Vorrichtung zu verringern.

5. Gerät gemäß Anspruch 4, wobei das mindestens eine Bild in zwei Dimensionen auf mindestens einer Oberfläche des Verankerungskörpers (20) dargestellt ist.

6. Gerät gemäß Anspruch 4, wobei das mindestens eine Bild in drei Dimensionen auf mindestens einer Oberfläche des Verankerungskörpers (20) dargestellt ist.

7. Gerät gemäß einem der vorherigen Ansprüche, wobei das Gerät Polyurethan, Urethan, Silikon oder eine Kombination davon umfasst.

8. Gerät gemäß einem der vorherigen Ansprüche, wobei der Verankerungskörper (20) die obere Oberfläche, die erste Seitenoberfläche und die zweite Seitenoberfläche der medizinischen Vorrichtung abdeckt, und wobei der Verankerungskörper, bei Abdeckung der medizinischen Vorrichtung, im Wesentlichen die gleiche Form aufweist wie die obere Oberfläche, die erste Seitenoberfläche und die zweite Seitenoberfläche der medizinischen Vorrichtung.

9. Gerät gemäß Anspruch 1, wobei das Gerät eine entfernbare Abdeckung ist.

10. System zum Befestigen einer angepassten dekorativen Abdeckung an einer intrakorporalen medizinischen Vorrichtung, welche eine obere Oberfläche, eine erste Seitenoberfläche, eine zweite Seitenoberfläche, einen Bodenoberfläche, ein Band, eine zentrale Öffnung und einen Schaft aufweist, wobei das System das Gerät gemäß Anspruch 1, ein Mittel zum Befestigen des Verankerungskörpers an der intrakorporalen medizinischen Vorrichtung, und ein Mittel zum Befestigen der Scharnierabdeckung an dem Band der intrakorporalen medizinischen Vorrichtung umfasst.

## Revendications

1. Appareil (10) pour recouvrir un dispositif médical intracorporel (80) ayant une surface supérieure, une première surface latérale (100), une seconde surface latérale (110), une surface inférieure, une attache (130), une ouverture centrale (140) et une tige (150), ledit dispositif médical intracorporel pouvant être inséré dans une ouverture artificielle pour faciliter la transmission de fluides dans le corps humain ou le retrait de fluides du corps humain à travers l'ouverture artificielle, l'appareil comprenant :
un corps d'ancrage (20) ayant une surface supérieure (30), une première surface latérale (40) et une seconde surface latérale (50), le corps d'ancrage étant adapté pour être en communication avec frottement avec une surface supérieure (90), une première surface latérale (100) et une seconde surface latérale (110) d'un dispositif médical intracorporel, **caractérisé en ce que** le corps d'ancrage (20) définit une ouverture pour l'insertion d'une attache (130) dans une ouverture centrale (140) du dispositif médical intracorporel ;
et un couvercle à charnière (60) relié au corps d'ancrage par un élément de liaison (70), le couvercle à charnière étant adapté pour être en communication avec l'attache (130) et entourer celle-ci de telle sorte que l'attache et l'ouverture centrale ne sont pas exposées à l'environnement extérieur après insertion de l'attache dans l'ouverture centrale (140) du dispositif médical.

2. Appareil selon la revendication 1, dans lequel, pendant l'utilisation, une tige (150) du dispositif médical intracorporel est insérée dans une ouverture artificielle.

3. Appareil selon la revendication 2, dans lequel le corps d'ancrage (20) n'est pas en communication avec une surface inférieure du dispositif médical intracorporel, ni en communication avec la tige (150) du dispositif médical intracorporel inséré dans l'ouverture artificielle, en outre dans lequel la surface inférieure du dispositif médical intracorporel n'est pas en communication avec le patient pendant l'utilisation et dans lequel le corps d'ancrage n'est pas en communication avec le patient pendant l'utilisation.

4. Appareil selon l'une quelconque des revendications précédentes, dans lequel l'appareil comprend au moins une image avec laquelle le patient a des associations psychologiques favorables, l'image étant adaptée pour encourager un patient à utiliser le dispositif médical intracorporel et réduire la crainte concernant l'utilisation du dispositif médical intracorporel.

5. Appareil selon la revendication 4, dans lequel la au moins une image est représentée en deux dimensions sur au moins une surface du corps d'ancrage (20).

6. Appareil selon la revendication 4, dans lequel ladite au moins une image est représentée en trois dimensions sur au moins une surface du corps d'ancrage (20).

7. Appareil selon l'une quelconque des revendications précédentes, dans lequel l'appareil comprend du polyuréthanne, de l'uréthanne, de la silicone ou une combinaison de ceux-ci.

8. Appareil selon l'une quelconque des revendications précédentes, dans lequel le corps d'ancrage (20) recouvre la surface supérieure, la première surface latérale et la seconde surface latérale du dispositif médical et, lors du recouvrement du dispositif médical, le corps d'ancrage a sensiblement la même forme que la surface supérieure, la première surface latérale et la seconde surface latérale du dispositif médical.

9. Appareil selon la revendication 1, dans lequel l'appareil est un couvercle amovible.

10. Système pour fixer un couvercle décoratif personnalisé à un dispositif médical intracorporel ayant une surface supérieure, une première surface latérale, une seconde surface latérale, une surface inférieure, une attache, une ouverture centrale et une tige, le système comprenant l'appareil de la revendication 1, des moyens pour fixer le corps d'ancrage au dispositif médical intracorporel, et des moyens pour fixer le couvercle à charnière à l'attache du dispositif médical intracorporel.
